# DEMANDE DE BREVET EUROPEEN

(11) **EP 3 767 271 A1**
(43) Date de publication de la demande: **20.01.2021**
(21) Numéro de dépôt: 20184548.4
(22) Date de dépôt: 07.07.2020
(51) Int. Cl.: G01N 1/04, B01L 3/00, G01N 33/53, G01N 33/72, G01N 33/543

(54) **PROCÉDÉ ET SYSTÈME DE DÉTECTION D'UN ANALYTE PRÉSENT DANS UN ÉCHANTILLON**

(30) Priorité: 17.07.2019 FR 1908035
(71) Demandeur: Commissariat à l'énergie atomique et aux énergies alternatives, 75015 Paris (FR)
(72) Inventeur: CUBIZOLLES, Myriam-Laure, 38054 GRENOBLE Cedex 09 (FR); BORDY, Thomas, 38054 GRENOBLE Cedex 09 (FR); REVOL-CAVALIER, Frédéric, 38054 GRENOBLE Cedex 09 (FR)
(74) Mandataire: INNOV-GROUP

(57) **Abrégé**

L'invention concerne un procédé de détection d'un analyte présent dans un échantillon liquide, caractérisé en ce qu'il comporte des étapes de :
- Injection dudit échantillon liquide dans une chambre de détection (23), ladite chambre de détection (23) présentant un volume non nul renfermant des billes (25) polymériques recouvertes d'un réactif adapté audit analyte à détecter,
- Capture d'au moins une image d'au moins une zone de la chambre de détection à l'aide d'un capteur (30),
- Traitement de ladite image acquise par le capteur, comprenant une détermination d'un niveau de texturation de ladite image acquise et une détermination d'une concentration dudit analyte en fonction du niveau de texturation déterminé pour ladite image.

## Description

### Domaine technique de l'invention

La présente invention se rapporte à un procédé et à un système de détection d'un analyte présent dans un échantillon liquide.

Le système sera notamment adapté pour détecter la présence d'hémoglobine humaine à partir d'un prélèvement de selles, pour le dépistage du cancer colorectal.

Le système sera également adapté à la détection d'analyte dans des prélèvements tels que :
- La boue pour la recherche de bactéries ou de champignons,
- Le sol pour la recherche de polluants, ou de composés particuliers (sulfates, fer, cuivre...),
- Des végétaux (par exemple champignons, salades, légumes, graines, fruits...) pour effectuer un suivi suite à une contamination (présence de pesticides, bactéries...),
- Des animaux, par exemple dans le cas d'une biopsie d'oreille sur du bétail.

On peut notamment citer la détection de présence de mycotoxines dans des graines ou de pesticides dans un environnement particulier (boue par exemple).

### Etat de la technique

Dans le domaine de la prévention du cancer colorectal, le dosage de l'hémoglobine est réalisé en laboratoire après un prélèvement de matière.

Les dispositifs de prélèvement peuvent comporter un outil de type écouvillon, bandelette, spatule... Ces outils sont maniés directement par l'utilisateur du test qui doit faire ses propres prélèvements et permettent de réaliser un prélèvement unitaire qui est calibré par la forme de l'outil. Une solution de prélèvement connue et disponible consiste à utiliser une canule. La canule est ensuite insérée dans un flacon muni d'un système de septum pour limiter l'excès de matière et ainsi mieux calibrer l'échantillon. Une autre solution connue consiste à employer un pot dans lequel les selles sont récupérées puis à réaliser un prélèvement par grattage à l'aide d'une canule. Dans le domaine des prélèvements de matière dans le sol, il existe également des systèmes permettant de réaliser un carottage calibré à l'aide d'un échantillonneur de terrain. Ces dispositifs connus sont constitués d'une tige creuse qui se remplit lorsqu'on l'enfonce dans le sol.

En ce qui concerne l'analyse, le dosage de l'hémoglobine fécale est réalisé sur un automate de laboratoire dédié, qui permet de mesurer optiquement par turbidimétrie (néphélométrie) l'immuno-agglutination de billes de latex greffées avec des anticorps dirigés contre l'hémoglobine humaine. L'inconvénient majeur de cette solution est que l'analyse ne peut être effectuée que plusieurs jours après le prélèvement. Cela implique une altération possible de l'hémoglobine lors du transport, et nécessite donc d'utiliser une solution de stabilisation de l'hémoglobine.

Il existe des systèmes portables de mesure de l'agrégation de microbilles pour le dosage de biomarqueurs. C'est le cas dans la publication référencée *"*Cui, W., He, M., Mu, L., Lin, Z., Wang, Y., Pang, W., Reed, M.A., and Duan, X. Cellphone-Enabled Microwell-based Microbead Aggregation Assay for Portable Biomarker Detection. ACS Sens. 2018, 3, 2, 432-440*".* Cette étude met en évidence un dosage quantitatif de la PSA ("Prostate Specific Antigen") avec des microbilles recouvertes d'anticorps anti-PSA et qui vont s'agréger en présence de PSA. Le système portable utilisé consiste à piéger ces microbilles agrégées au sein de puits contenus dans une puce micro-fluidique et à mesurer optiquement, à l'aide d'un smartphone interfacé à un microscope optique portable, le nombre de billes agrégées contenues dans les puits. L'inconvénient majeur de cette solution réside dans la préparation de l'échantillon qui doit être réalisée manuellement en dehors du système.

D'autres études font également état de l'utilisation directe de smartphones et de la lumière ambiante pour détecter des micro-objets, tels que des billes ou des cellules animales. C'est le cas dans la publication référencée *"*Lee, S. A.; Yang, C. A Smartphone-Based Chip-Scale Microscope Using Ambient Illumination. Lab. Chip 2014, 14 (16), 3056-3063*".*

Le but de l'invention est de proposer une solution permettant de réaliser un dosage quantitatif ou semi-quantitatif d'un analyte, de manière :
- simple,
- directe et rapide, c'est-à-dire sur site, sans transfert de prélèvement vers un laboratoire et sans employer un matériel trop encombrant, et
- fiable.

### Exposé de l'invention

Ce but est atteint par un procédé de détection d'un analyte présent dans un échantillon liquide, caractérisé en ce qu'il comporte des étapes de :
- Positionnement d'un support incluant une chambre de détection sur un dispositif de préparation par carottage,
- Préparation par carottage d'un échantillon solide et dissolution ou re-suspension dudit échantillon solide en vue de l'obtention d'un échantillon liquide,
- Injection dudit échantillon liquide dans ladite chambre de détection, ladite chambre de détection présentant un volume non nul renfermant des billes polymériques recouvertes d'un réactif adapté audit analyte à détecter, permettant une réaction d'agglutination en présence de l'analyte,
- Capture d'au moins une image d'au moins une zone de la chambre de détection à l'aide d'un capteur,
- Traitement de ladite image acquise par le capteur, comprenant une détermination d'un niveau de texturation de ladite image acquise, ledit niveau de texturation dépendant du niveau d'agglutination des billes polymériques en présence de l'analyte, et une détermination d'une concentration dudit analyte en fonction du niveau de texturation déterminé pour ladite image.

Selon une particularité, l'étape de capture est réalisée par imagerie défocalisée.

L'invention concerne également un système de détection d'un analyte présent dans un échantillon liquide, comprenant :
- Un support doté d'un circuit fluidique, ledit circuit fluidique comportant au moins un canal d'injection et une chambre de détection dans laquelle débouche ledit canal d'injection, ladite chambre de détection présentant un volume non nul renfermant des billes polymériques recouvertes d'un réactif adapté audit analyte à détecter,
- Un dispositif de lecture optique, comprenant un capteur d'image agencé pour acquérir une image d'au moins une zone de la chambre de détection,
- Une unité de traitement configurée pour traiter chaque image acquise par le capteur, comprenant un module de détermination d'un niveau de texturation de chaque image acquise et un module de détermination d'une concentration dudit analyte en fonction du niveau de texturation déterminé pour chaque image, ledit niveau de texturation dépendant du niveau d'agglutination des billes polymériques dans la chambre de détection en présence de l'analyte.

Selon une particularité, le capteur et la zone de la chambre de détection sont positionnés de manière défocalisée.

Selon une autre particularité, le dispositif de lecture optique comporte une source de rayonnement lumineux agencé pour émettre un rayonnement lumineux à travers la chambre de détection, ledit capteur étant positionné de l'autre côté de ladite source par rapport à la zone de la chambre de détection.

Selon une autre particularité, le support est constitué d'une carte micro-fluidique réalisée dans un matériau transparent ou semi-transparent.

Selon une autre particularité, le système comporte un dispositif de préparation de l'échantillon liquide à analyser sur lequel est adapté ledit support, ledit dispositif de préparation comprenant un étage de carottage d'un prélèvement de matière et un étage de dissolution ou de re-suspension de chaque carotte réalisé par ledit étage de carottage, auquel est relié ledit canal d'injection du circuit fluidique.

Selon une autre particularité, l'étage de dissolution ou de re-suspension comporte une ou plusieurs cuvettes destinées chacune à recevoir un liquide de dissolution ou de re-suspension.

Selon une autre particularité, le dispositif de préparation comporte un corps et en ce que l'étage de carottage comporte un ou plusieurs emporte-pièces mobiles par rapport au corps, chaque emporte-pièce coopérant en translation avec ledit corps suivant une direction principale, entre au moins un état de repos dans lequel l'emporte-pièce est à l'aplomb d'une surface de dépôt destiné à recevoir ledit prélèvement de matière et un état actionné dans lequel l'emporte-pièce est situé à l'intérieur d'une cuvette distincte de l'état de dissolution ou de re-suspension.

Selon une autre particularité, chaque cuvette comporte une ouverture et en ce qu'il comporte au moins un opercule perforable recouvrant l'ouverture de chaque cuvette.

Selon une autre particularité, le circuit fluidique comporte une zone de filtration des particules présentes dans l'échantillon liquide.

Selon une autre particularité, le dispositif de lecture optique et l'unité de traitement sont regroupés dans un même dispositif de type téléphone intelligent.

### Brève description des figures

D'autres caractéristiques et avantages vont apparaître dans la description détaillée qui suit faite en regard des dessins annexés dans lesquels :
- La figure 1 représente de manière schématique le système de l'invention.
- Les figures 2A à 2C représentent, de manière schématique, une première configuration d'un dispositif de préparation pouvant être employé dans le système de l'invention, dans trois états de fonctionnement différents.
- La figure 3 représente, de manière schématique, une deuxième configuration d'un dispositif de préparation pouvant être employé dans le système de l'invention, à l'état repos.
- La figure 4 représente, de manière schématique et en vue de dessus, la carte micro-fluidique pouvant être employé dans le système de l'invention.
- Les figures 5A et 5B illustrent un principe de connexion fluidique de la carte micro-fluidique.
- La figure 6 illustre le principe de détection mis en œuvre par le système de l'invention.
- La figure 7 illustre un principe de microscopie en réflexion pouvant être employé dans le système de l'invention.
- Les figures 8A et 8B représentent deux images acquises par le capteur du système de l'invention, respectivement à t=0s et à t=60 minutes.
- Les figures 9A et 9B représente respectivement un diagramme de variation de l'erreur standard en fonction du temps pour différentes concentrations d'hémoglobine et un diagramme de variation de l'erreur standard normalisé en fonction de la concentration d'hémoglobine, à t=40minutes.

### Description détaillée d'au moins un mode de réalisation

Pour la suite de la description et sur les figures annexées, on définit un axe principal (A) suivant une direction verticale, perpendiculaire à la surface sur laquelle est posé le système de l'invention. Cette surface peut notamment être le sol ou tout autre support plan.

Les termes "supérieur", "inférieur", "dessus", "dessous" ou équivalents utilisés ci-dessous dans la description sont à comprendre en se référant audit axe principal (A).

L'invention vise à permettre le dosage d'un analyte en utilisant le principe de l'agglutination de billes (nanométrique ou micrométrique) polymériques (par exemple en latex). L'invention peut être utilisée dans n'importe quel domaine, pour différents types de prélèvement, à partir du moment où le prélèvement peut être re-suspendu ou solubilisé en milieu liquide.

Cette invention trouve sa place dans tout domaine où il sera possible de réaliser un dosage quantitatif ou semi-quantitatif d'une cible d'intérêt par l'utilisation de l'agglutination de micro ou nano-billes. Ces billes, de taille micrométrique ou le plus souvent nanométrique, sont recouvertes d'un réactif adapté (par exemple anticorps) qui reconnait la cible. En présence de ce dernier, un pontage est réalisé entre les différentes billes : il s'agit d'une réaction d'agglutination, par exemple d'immuno-agglutination. Plus la quantité de cible est importante, plus l'agrégation des billes entre elles sera importante. On verra qu'une mesure optique de diffusion de la lumière permet ensuite de quantifier précisément la concentration de la cible dans l'échantillon analysé.

L'invention est notamment adaptée à la détection de la présence d'hémoglobine humaine dans les selles pour l'amélioration du test de dépistage du cancer colorectal.

Comme indiqué ci-dessus, on peut également citer la détection de présence de mycotoxines dans des graines ou de pesticides dans un environnement particulier (boue par exemple).

Le système de l'invention comporte entre autres une architecture de détection optique et une unité de traitement intégrées lui permettant d'être employé directement sur le terrain.

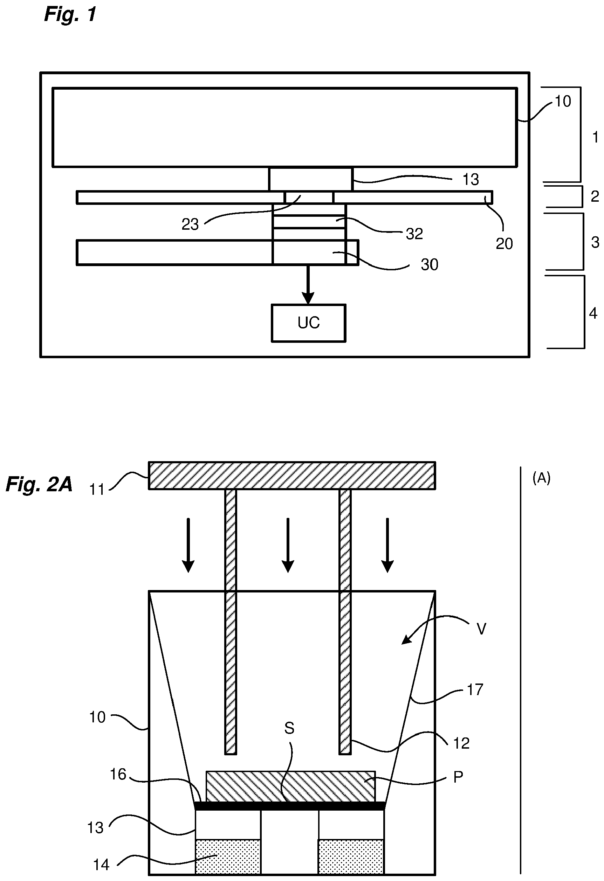

En référence à la figure 1, le système de l'invention présente avantageusement une architecture en plusieurs parties :
- Une première partie 1 composée d'un dispositif de préparation d'un échantillon calibré à partir d'un prélèvement de matière ;
- Une deuxième partie 2 composée d'une carte micro-fluidique dotée d'une chambre de détection dans laquelle sont placées les billes décrites ci-dessus pour réaliser la réaction d'agglutination ;
- Une troisième partie 3 composée d'un dispositif de lecture optique comprenant notamment un capteur pour capturer des images de la chambre de détection ;
- Une quatrième partie 4 composée d'une unité de traitement configurée pour traiter les images acquises par le capteur ;

Le système présente avantageusement une architecture monobloc, comprenant un corps, réalisé par exemple en matériau plastique, sur lequel peuvent venir se connecter différents éléments nécessaires au fonctionnement du système.

La première partie 1 du système, formée d'un dispositif de préparation de l'échantillon, peut présenter différentes configurations. Ce dispositif de préparation a un double objectif : Réaliser au moins un échantillon calibré à partir d'un prélèvement de matière et assurer une dissolution ou re-suspension de cet échantillon calibré dans un liquide pour pouvoir être transféré dans la carte micro-fluidique dotée de la chambre de détection.

Il faut comprendre que le système de l'invention n'est pas limité au dispositif de préparation qui est décrit ci-dessous et qu'il peut fonctionner avec un dispositif de préparation présentant des caractéristiques différentes. Le système requiert une solution pour obtenir au moins un échantillon calibré et le dissoudre ou re-suspendre dans un liquide afin de pouvoir l'injecter dans la carte micro-fluidique pour analyse.

De manière avantageuse, le dispositif de préparation employé peut permettre de préparer plusieurs échantillons calibrés de matière de manière simultanée à partir d'un même prélèvement P de matière (avantageusement molle). De manière non limitative, le dispositif pourra permettre la préparation de un à dix échantillons calibrés à partir d'un même prélèvement. Sur les figures annexées, la représentation schématique du dispositif montre la préparation simultanée de deux échantillons distincts à partir d'un même prélèvement P de matière.

A titre d'exemple, la matière pourra être des selles d'un être vivant (notamment humain), de la boue, de la matière présente dans le sol. Mais le prélèvement P pourra également être de type végétal (des graines de tailles différentes) ou d'origine animale.

Dans le cas de selles d'origine humaine, les échantillons générés pourront notamment servir à détecter la présence d'hémoglobine pour dépister le cancer colorectal.

De manière non limitative, on peut considérer que la matière du prélèvement P qui sert de base à la préparation des échantillons doit être suffisamment molle pour être carottée par la seule force de l'utilisateur. Pour les selles, la dureté du prélèvement pourra être comprise entre 1 et 7 sur l'échelle de Bristol. Cependant, pour certains prélèvements plus durs (comme par exemple des grains durs), il est possible d'utiliser le dispositif uniquement pour calibrer un échantillon (sans découpage/carottage du prélèvement), notamment dans le cas où la taille du prélèvement s'avère plus réduite que la taille de l'emporte-pièce qui est employé dans le dispositif.

Le dispositif de préparation peut se présenter sous la forme d'un ensemble monobloc, facilement transportable.

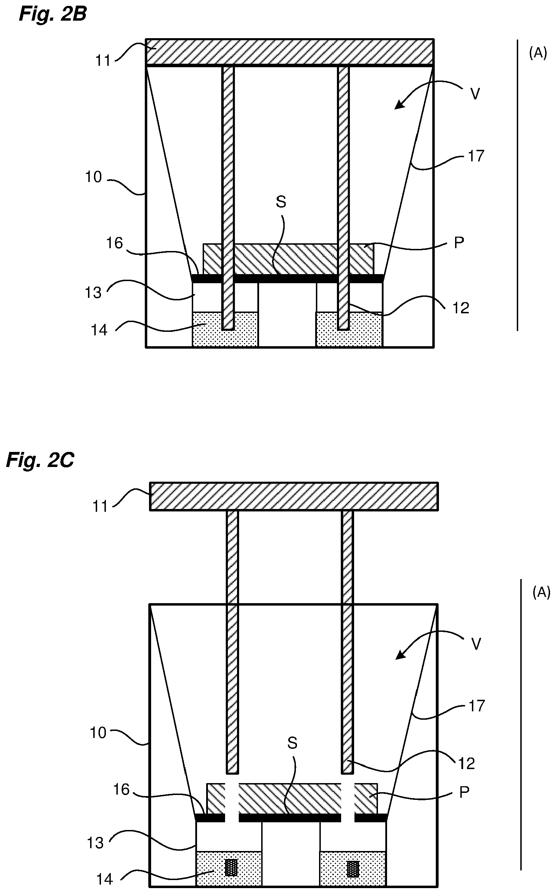

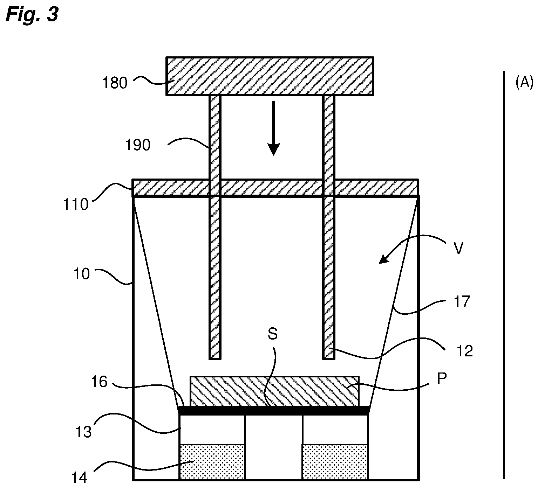

Les figures 2A à 2C représentent une première configuration du dispositif de préparation et la figure 3 représente une deuxième configuration du dispositif de préparation.

Dans ces deux configurations, le dispositif de préparation peut comporter un corps définissant un espace interne V formé par l'assemblage d'un réceptacle 10 et d'un couvercle 11, 110 venant se positionner sur le réceptacle pour le refermer. Le réceptacle est avantageusement destiné à être posé sur le sol ou sur tout support approprié. Lorsque le couvercle 11, 110 est apposé sur le réceptacle, l'espace interne V est avantageusement isolé de l'extérieur de manière hermétique.

De manière non limitative, le réceptacle 10 peut être de forme cylindrique, présentant un fond, une ou plusieurs parois latérales et une ouverture supérieure destinée à être obturée par le couvercle. Bien entendu, toute autre forme pourrait être adaptée.

Le dispositif peut comporter un support réalisé sous la forme d'une plateforme logée dans l'espace interne V du réceptacle. La plateforme comporte une surface de dépôt S du prélèvement à partir duquel est préparé un ou plusieurs des échantillons. Cette surface de dépôt S est avantageusement plane et orientée perpendiculairement à l'axe principal (X).

La plateforme comporte un opercule 16 perforable dont la surface supérieure constitue la surface de dépôt S du prélèvement. De manière non limitative, l'opercule peut être réalisé sous la forme d'un film plastique, polymère, élastomère (par exemple une mousse) ou métallique venant recouvrir de manière hermétique une ou plusieurs cuvettes 13 situées sous la plateforme. Il peut également être réalisé sous la forme d'un élément multicouches employant plusieurs des matériaux cités ci-dessus. La perforation est rendue possible par un simple appui contre la surface de l'opercule suivant une direction transversale à ladite surface S, préférentiellement normale à ladite surface. Cette direction de perforation est donc avantageusement parallèle à l'axe principal (A) défini ci-dessus. On verra que l'appui permettant la perforation de l'opercule 16 est mis en œuvre à l'aide d'au moins un emporte-pièce 12 du dispositif.

Les cuvettes forment un étage de dissolution ou re-suspension des échantillons. Chaque cuvette 13 est en effet destinée à recevoir un liquide 14 choisi pour permettre la dissolution ou la re-suspension de l'échantillon préparé. Une cuvette 13 distincte est avantageusement dédiée à un échantillon particulier préparé à partir du prélèvement. Le nombre de cuvettes n'est pas limitatif. Il pourra être compris entre un et dix, comme le nombre d'échantillons à préparer ou même être supérieur au nombre d'échantillons à préparer. Dans ce dernier cas, les cuvettes supplémentaires pourront servir d'étalon ou de contrôle, par exemple pour conserver une référence et élaborer un suivi du vieillissement dans le temps des solutions embarquées dans les cuvettes.

Le dispositif de préparation de l'invention comporte un étage de carottage destiné à créer un ou plusieurs échantillons à partir du prélèvement P de matière déposé sur la surface de dépôt S de la plateforme.

Pour chaque échantillon calibré à préparer, l'étage de carottage comporte un emporte-pièce 12 distinct destiné à découper le prélèvement P de matière pour réaliser un échantillon calibré.

De manière avantageuse, l'étage de carottage comporte plusieurs emporte-pièces 12 en parallèle, permettant de préparer plusieurs échantillons de manière simultanée à partir d'un même prélèvement P.

L'étage de carottage est agencé de manière à permettre une translation des emporte-pièces 12 suivant une direction donnée, normale par rapport à la surface de dépôt S et donc parallèle à l'axe principal (X).

Chaque emporte-pièce 12 peut être réalisé sous la forme d'une partie creuse à l'extrémité libre d'un tube, la partie creuse du tube définissant le volume de la carotte créée lors de la préparation de l'échantillon et donc le volume de l'emporte-pièce. Les tubes sont disposés parallèles entre eux et sont arrangés de sorte que leur axe est parallèle à l'axe principal (A). Chaque tube présente une extrémité fixée à une partie mobile du dispositif (couvercle ou ensemble mobile) et son extrémité libre qui comporte la partie creuse formant l'emporte-pièce. Bien entendu, toute autre forme pourrait être adaptée, à partir du moment où l'emporte-pièce permet de réaliser un carottage du prélèvement.

De manière particulière, dans la première configuration représentée sur les figures 2A à 2C, les emporte-pièces 12 sont directement portés par le couvercle 11 du dispositif. Ils sont alors orientés vers l'intérieur du réceptacle 10 lorsque le couvercle 11 est apposé sur le réceptacle 10 pour le refermer. Un mécanisme de glissière peut être prévu entre le réceptacle 10 et le couvercle 11 pour guider le positionnement du couvercle 11 sur le réceptacle 10 suivant la direction de l'axe principal (A). En venant apposer le couvercle sur le réceptacle suivant le mouvement de translation, chaque emporte-pièce 12 vient découper une partie du prélèvement.

Dans la deuxième configuration représentée sur la figure 3, l'étage de carottage peut comporter un mécanisme mobile coopérant en coulissement avec le corps du dispositif. De manière non limitative, ce mécanisme mobile peut comporter un organe de préhension 180 situé à l'extérieur du corps du dispositif, le rendant ainsi accessible pour une prise manuelle. Cet organe de préhension 180 peut se présenter sous la forme d'une poignée. Le mécanisme mobile comporte un ensemble mobile en translation dans l'espace interne V du corps du dispositif. Ledit organe de préhension 180 est relié à cet ensemble mobile par l'intermédiaire d'au moins une tige de liaison 190. Ladite tige de liaison est agencée pour traverser le corps du dispositif, à travers au moins une ouverture, formant ainsi un mécanisme de glissière entre le mécanisme mobile et le corps du dispositif. La tige de liaison 190 permet de transmettre le mouvement de translation effectué sur l'organe de préhension à l'ensemble mobile. La liaison peut être réalisée à travers le couvercle 110 du dispositif, qui est alors fixe et qui fait partie du corps du dispositif. Comme évoqué ci-dessus, l'ensemble mobile comporte alors un ou plusieurs emporte-pièces 12 destinés chacun à découper une partie du prélèvement P disposé sur la surface de dépôt S.

Dans ces deux configurations, on peut distinguer deux états distincts du dispositif de préparation :
- Un état de repos dans lequel les emporte-pièces 12 ont leur extrémité libre située à l'aplomb du prélèvement P disposé sur la surface de dépôt S. Autrement dit, dans cet état de repos, l'extrémité libre de chaque emporte-pièce 12 est située au-dessus du prélèvement. Dans la première configuration évoquée ci-dessus, le couvercle 11 n'est donc pas refermé sur le réceptacle. Dans la deuxième configuration, l'ensemble mobile est relevé et l'organe de préhension 180 émerge par rapport au couvercle 110.
- Un état actionné dans lequel l'extrémité libre de chaque emporte-pièce 12 vient se loger dans une cuvette 13 correspondante. Dans la première configuration, le couvercle 11 vient alors refermer le réceptacle. Dans la deuxième configuration, l'ensemble mobile est translaté vers le bas par pression sur l'organe de préhension 180 suivant l'axe principal (A).

Entre ses deux états, chaque emporte-pièce 12 est amené d'abord à traverser le prélèvement P pour le découper puis à perforer l'opercule 16 recouvrant une ou plusieurs cuvettes. Si le prélèvement P ne peut pas être découpé (car trop petit par exemple dans le cas d'une graine), l'emporte-pièce 12 permet de calibrer l'échantillon.

Comme déjà décrit, chaque cuvette 13 est destinée à être remplie d'un liquide, celles qui sont en vis-à-vis d'un emporte-pièce permettant la dissolution ou la re-suspension de l'échantillon.

La deuxième partie 2 du système comporte pour sa part une unique carte micro-fluidique 20 destinée à recevoir le liquide dans laquelle chaque échantillon calibré a été dissolu ou re-suspendu.

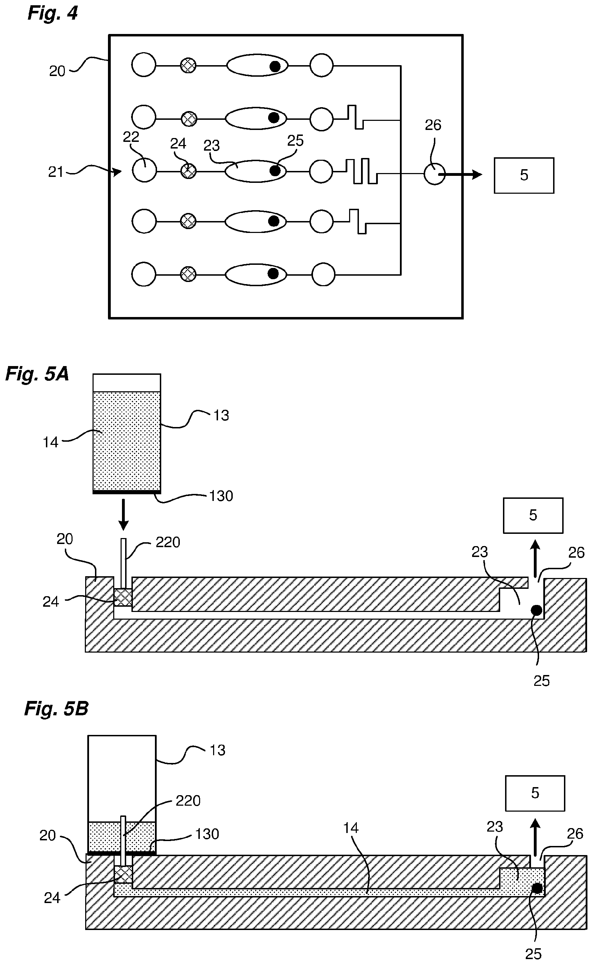

Comme représenté sur la figure 4, de manière classique, la carte micro-fluidique 20 peut se présenter sous la forme d'un support plan formé de plusieurs couches superposées collées entre elles. La carte 20 comporte un ou plusieurs circuits fluidiques 21, formant chacun une voie fluidique d'analyse distincte. Un circuit fluidique 21 peut être réalisé par une empreinte creusée (par exemple par gravure, moulage ou usinage) sur une face d'une couche de la carte 20 et recouverte par une autre couche de la carte.

De manière non limitative, la carte 20 peut comporter au moins autant de circuits fluidiques 21 qu'il y a de cuvettes dans le dispositif de préparation. Sur la figure 4, à titre d'exemple, la carte 20 comporte cinq circuits fluidiques identiques en parallèle. La carte 20 peut également comporter un ou plusieurs circuits-fluidiques dits de référence. Ces circuits fluidiques de référence peuvent notamment remplacer les cuvettes dites de référence décrites ci-dessus et employées pour le suivi du vieillissement des solutions embarquées.

Le corps du système peut comporter un logement dans lequel peut être insérée la carte micro-fluidique 20. La carte micro-fluidique 20 se présente ainsi comme une cartouche amovible et jetable par rapport au reste du système. L'insertion de la carte 20 dans le logement permet d'enclencher une connexion fluidique étanche entre une sortie fluidique reliée à chaque cuvette 13 du dispositif de préparation et une entrée fluidique 22 distincte de chaque circuit fluidique réalisé sur la carte 20.

Sur la carte micro-fluidique, chaque entrée fluidique est reliée par un canal d'entrée à une chambre de détection 23 formée d'une cavité de volume non nulle, de forme évasée par rapport à la section du canal d'entrée.

Comme représenté sur les figures 5A et 5B, de manière non limitative, chaque entrée fluidique peut être formée d'un canal réalisé dans une aiguille 220 creuse destinée à s'interfacer avec une cuvette 13 du dispositif de préparation. La cuvette du système de prélèvement, qui contient le liquide permettant la re-suspension de l'échantillon, peut comporter à sa base un septum 130 ou un opercule perforable par l'aiguille 220, par exemple au moment de l'insertion de la carte micro-fluidique 20 dans son logement. En variante, la connexion fluidique peut être assurée par un embout venant se connecter à la cuvette par l'intermédiaire d'une valve munie d'un joint (par exemple un joint torique) permettant d'assurer l'étanchéité. Toute autre solution peut bien entendu être envisagée.

Le remplissage de chaque circuit fluidique 21 peut ensuite être réalisé par gravité, ou en employant une source de dépression comme décrit ci-après.

Entre son entrée et sa chambre de détection, chaque circuit fluidique 21 peut comporter une zone de filtration 24 destinée à filtrer des particules gênantes pour la suite de l'analyse optique. Il peut s'agir par exemple de débris de particules fécales dans le cas d'un prélèvement de selles, ou alors de cellules sanguines (globules rouges et globules blancs) dans le cas d'un prélèvement sanguin. A titre d'exemple, la zone de filtration 24 peut être composée d'un réservoir, contenant le ou les matériau(x) permettant de réaliser la filtration. Il peut s'agir par exemple de cellulose, de polyuréthane expansé ou encore de polymère. Les matériaux peuvent être combinés entre eux sous forme de couche, de billes ou de gradient si nécessaire.

En aval de sa zone de filtration, le circuit fluidique comporte sa chambre de détection 23. Cette chambre de détection 23 est destinée à contenir les nano-billes 25 recouvertes d'un réactif adapté (par exemple anticorps) nécessaires à la détection de la cible d'intérêt. Ces billes 25 sont embarquées sous forme séchée ou lyophilisée dans la chambre 23 et sont maintenues au sein de la chambre, par exemple à l'aide d'un filet ("mesh" de type grille ou filtre hydrophobe) doté d'une maille de taille adaptée. Il faut noter que la chambre de détection 23 est dimensionnée pour que l'acquisition d'images par le capteur (de type CMOS) du dispositif de lecture puisse être effectuée. En particulier, l'épaisseur de la chambre, ainsi que le ou les matériau(x) la composant sont des critères importants à prendre en compte.

La carte micro-fluidique 20 peut comporter un ou plusieurs évents. L'évent 26 peut être commun à l'ensemble des circuits fluidiques. Il est également possible de prévoir un évent distinct pour chaque circuit fluidique. Chaque évent peut être réalisé par une simple ouverture sur l'extérieur. De manière non limitative, l'évent 26 peut également être connecté à une source de dépression permettant de commander le remplissage de chaque circuit fluidique 21. Cette source de dépression peut être constituée d'une seringue 5 ou de toute autre solution de pompage.

Au niveau de chaque chambre de détection 23, la carte est réalisée dans un matériau transparent. Par le terme "transparent", on entend que le matériau employé est au moins partiellement transparent à la lumière visible, de manière à laisser passer au moins 80% de cette lumière. Il faut ainsi comprendre qu'il sera suffisamment transparent pour voir l'intérieur de la chambre 23.

L'ensemble de la carte 20 peut être réalisé dans un matériau transparent ou semi-transparent de type PMMA (Polyméthacrylate de Méthyle), COC ou équivalent. La cartouche insérée dans le système présente avantageusement une configuration (notamment fluidique) qui est toujours identique de manière à coopérer avec les éléments du système qui sont nécessaires à la mise en œuvre de l'analyse. Le matériau choisi sera le moins adsorbant possible pour éviter que les molécules ciblées (hémoglobine) viennent se coller sur le support.

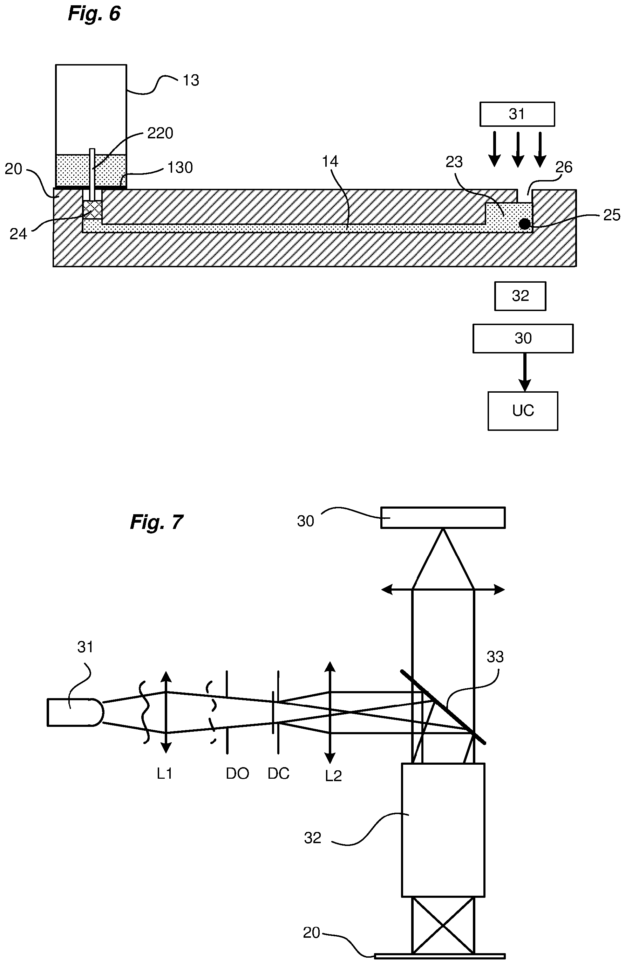

En référence à la figure 6, la troisième partie 3 du système est composée du dispositif de lecture optique permettant de réaliser le suivi de la réaction d'agglutination au sein de chaque chambre de détection 23. La lecture optique peut être réalisée à travers une zone de lecture restreinte de chaque chambre de détection et non sur toute la chambre de détection. Le dispositif de lecture optique peut être unique et commun à toutes les chambres de détection 23. Le dispositif de lecture optique comporte au moins un capteur 30 et optionnellement au moins une source lumineuse 31. La source lumineuse 31 peut être une diode électroluminescente. Elle est agencée pour illuminer la chambre de détection du circuit fluidique 21. Le capteur 30 peut être de type CMOS. Il peut notamment s'agir du capteur présent sur un téléphone intelligent ("smartphone"). Optionnellement, le dispositif de lecture optique peut comporter une lentille 32 de grossissement adaptée, intercalée entre le capteur 30 et la chambre de détection 23. Lorsqu'un seul dispositif est employé pour plusieurs chambres de détection, il est possible de prévoir des moyens mécaniques d'actionnement, commandés pour positionner chaque chambre de détection entre le capteur 30 et la source lumineuse 31. Ces moyens peuvent agir sur la carte micro-fluidique 20 ou sur le dispositif de lecture optique pour opérer un déplacement relatif de l'un par rapport à l'autre. Une séquence de déplacement peut être prévue pour balayer toutes les chambres de détection du système. Si le capteur 30 est intégré à un téléphone intelligent, la source lumineuse 31 peut être optionnelle.

On peut noter que des résultats peuvent être obtenus en employant un principe d'imagerie de diffraction sans lentille ou avec lentille (un téléphone intelligent intègre déjà une lentille). Avantageusement, la zone de lecture de la chambre et le capteur sont positionnés de manière défocalisée pour obtenir des images de diffraction des cibles qui sont plus contrastées, de manière à pouvoir mieux les distinguer.

Pour capturer les images, il est notamment possible d'employer un système de microscopie en réflexion, comme illustré par la figure 7. Ce système comporte une solution d'éclairement de la carte 20 portant l'échantillon, composée de la source lumineuse 31, de lentilles L1, L2, de diaphragmes DO, DC et d'une lame réfléchissante 33. La lentille 32 de grossissement est positionnée entre la lame 33 et la carte 20. Le capteur 30 est positionné au-dessus de la lame 33 pour capturer des images de la zone de lecture de la carte 20.

Le capteur 30 est destiné à capturer des images successives de la zone de lecture de la chambre de détection 23, permettant ainsi de suivre l'évolution de la réaction d'agglutination.

La quatrième partie 4 du système est composée de l'unité de traitement UC chargée de traiter les images acquises par le capteur 30. Cette unité de traitement UC peut être celle déjà présente dans le téléphone intelligent dont le capteur CMOS a servi pour capturer les images de la chambre de détection. Les images acquises sont alors stockées dans une mémoire du téléphone.

L'unité de traitement UC est destinée à déterminer la concentration de l'analyte d'intérêt à partir des images de la chambre de détection qui ont été acquises par le capteur 30 puis mémorisées.

L'acquisition des images est effectuée au cours d'intervalles de temps définis durant un temps défini. Au cours de cette cinétique, l'aspect des images acquises va changer, notamment la texturation, due à l'agglutination des nano-billes 25 en présence de la cible d'intérêt que l'on souhaite doser.

L'utilisation d'un indicateur simple (par ex. variation de l'erreur standard du niveau de gris d'une zone d'intérêt de l'image mesurée au cours du temps) permet ensuite de corréler la mesure à la quantité de cible à doser.

Pour cela l'unité de traitement UC comporte ainsi un module de détermination d'un niveau de texturation de chaque image acquise et un module de détermination d'une concentration dudit analyte en fonction du niveau de texturation déterminé pour chaque image.

### Exemple de traitement mis en œuvre expérimentalement :

Des essais ont été réalisés pour la détection de l'hémoglobine humaine à l'aide de nano-billes de latex greffées avec un anticorps ("OC Sensor"). Pour cela, une solution de selles de "mini-pigs" a été préparée dans une solution tampon commerciale et supplémentée avec une gamme d'hémoglobine humaine comprise entre 0 et 500ng/mL. Cette solution est mélangée avec la solution tampon réactionnelle et les billes de latex. Puis quelques microlitres de chaque échantillon (simulant un prélèvement) sont rapidement placés dans la chambre de détection micro-fluidique (ex : 300µm d'épaisseur). Cette chambre de détection est placée en vis-à-vis du capteur de type CMOS (ex : CMOS 1.67 µm, 30 mm²), et est illuminée par une diode électroluminescente via un orifice ("pinhole") (ex : DEL 450nm fibrée 400µm). Pour certains des essais, une lentille est ajoutée au dispositif (ex : objectif x20 + lentille tube x0.3, conduisant à un grossissement x6). Le suivi de la cinétique d'agglutination est réalisé par des acquisitions d'images à des intervalles de temps réguliers (par exemple : toutes les 5 minutes). Une région d'intérêt (ROI " "Région Of Interest") est ensuite définie au sein de la série d'images et un indicateur pertinent du niveau de gris de l'image (par exemple : erreur standard, Kurtosis) est ensuite choisi afin de comparer les images les unes par rapport aux autres au niveau de la région d'intérêt sélectionnée. Cet indicateur reflète directement la variation de l'agglutination en fonction du temps.

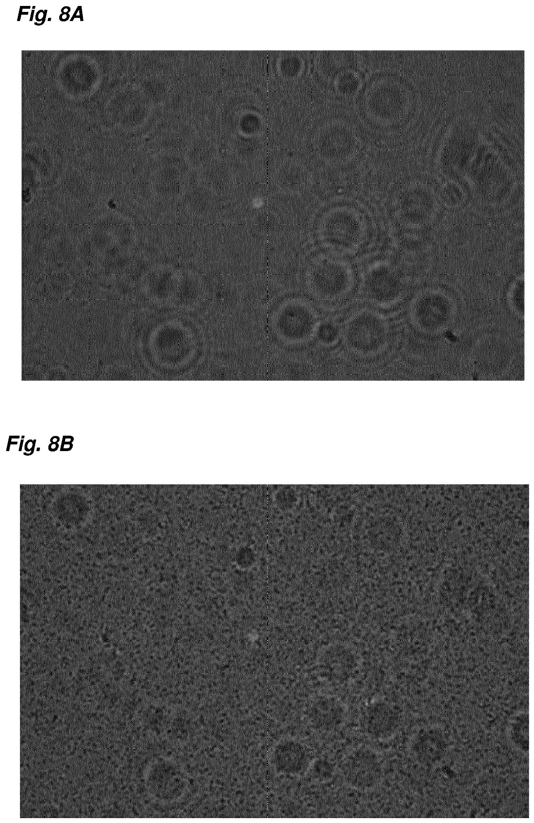

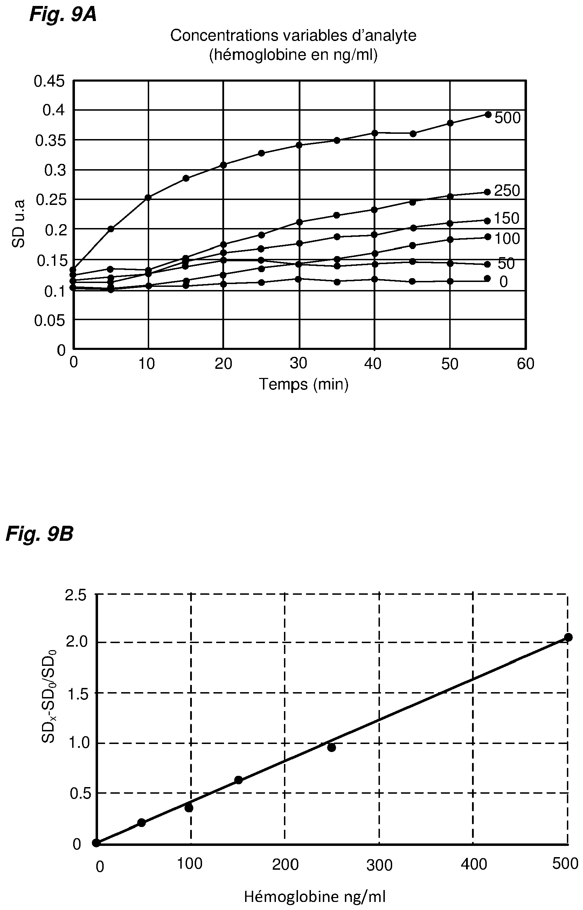

Des images obtenues avec un principe d'imagerie défocalisée avec lentille mettent en évidence un changement de texturation au cours du temps, caractéristique de la réaction d'agglutination qui est en train de se dérouler. Les figures 8A et 8B représentent ainsi les images obtenues au bout de 0min et 60min de réaction d'agglutination avec une solution de selles de "mini-pigs". Le graphique de la figure 9A présente la variation de la valeur de l'erreur standard (SD) du niveau de gris de la région d'intérêt en fonction du temps. Ce premier indicateur de texture suffit à quantifier la réaction. Le graphique de la figure 9B illustre la valeur de l'indicateur SDₓ (erreur standard - "standard déviation") normalisé par rapport au SD₀ au bout de 40 minutes de réaction en fonction de la concentration en hémoglobine : une relation linéaire est alors établie.

On comprend de ce qui précède que la solution de l'invention est particulièrement avantageuse :
- Le système complet inclut les moyens de préparation de l'échantillon, le support de détection et le dispositif de lecture et de traitement, dans une architecture particulièrement compacte, le rendant facilement transportable sur le terrain ;
- Le système est facile à employer car il utilise des moyens mécaniques, électroniques et logiciels particulièrement simples et efficaces ;
- Le système est efficace car il permet de déterminer une concentration d'hémoglobine par simple traitement d'images ; Il ne requiert donc pas l'emploi de matériels encombrants et onéreux ;

## Revendications

1. Procédé de détection d'un analyte présent dans un échantillon liquide, **caractérisé en ce qu'**il comporte des étapes de :
- Positionnement d'un support incluant une chambre de détection sur un dispositif de préparation par carottage,
- Préparation par carottage d'un échantillon solide et dissolution ou re-suspension dudit échantillon en vue de l'obtention d'un échantillon liquide,
- Injection dudit échantillon liquide dans ladite chambre de détection (23), ladite chambre de détection (23) présentant un volume non nul renfermant des billes (25) polymériques recouvertes d'un réactif adapté audit analyte à détecter, permettant une réaction d'agglutination en présence de l'analyte,
- Capture d'au moins une image d'au moins une zone de la chambre de détection à l'aide d'un capteur (30),
- Traitement de ladite image acquise par le capteur, comprenant une détermination d'un niveau de texturation de ladite image acquise, ledit niveau de texturation dépendant du niveau d'agglutination des billes polymériques en présence de l'analyte, et une détermination d'une concentration dudit analyte en fonction du niveau de texturation déterminé pour ladite image.

2. Procédé selon la revendication 1, **caractérisé en ce que** l'étape de capture est réalisée par imagerie défocalisée.

3. Système de détection d'un analyte présent dans un échantillon liquide, comprenant :
- Un support doté d'un circuit fluidique, ledit circuit fluidique (21) comportant au moins un canal d'injection et une chambre de détection (23) dans laquelle débouche ledit canal d'injection, ladite chambre de détection (23) présentant un volume non nul renfermant des billes (25) polymériques recouvertes d'un réactif adapté audit analyte à détecter,
- un dispositif de préparation de l'échantillon liquide à analyser sur lequel est adapté ledit support, ledit dispositif de préparation comprenant un étage de carottage d'un prélèvement de matière et un étage de dissolution ou de re-suspension de chaque carotte réalisé par ledit étage de carottage, auquel est relié ledit canal d'injection du circuit fluidique,
**Caractérisé en ce qu'**il comporte :
- Un dispositif de lecture optique, comprenant un capteur (30) d'image agencé pour acquérir une image d'au moins une zone de la chambre de détection (23),
- Une unité de traitement (UC) configurée pour traiter chaque image acquise par le capteur (30), comprenant un module de détermination d'un niveau de texturation de chaque image acquise et un module de détermination d'une concentration dudit analyte en fonction du niveau de texturation déterminé pour chaque image, ledit niveau de texturation dépendant du niveau d'agglutination des billes polymériques dans la chambre de détection en présence de l'analyte.

4. Système selon la revendication 3, **caractérisé en ce que** le capteur et la zone de la chambre de détection sont positionnés de manière défocalisée.

5. Système selon la revendication 3 ou 4, **caractérisé en ce que** le dispositif de lecture optique comporte une source (31) de rayonnement lumineux agencé pour émettre un rayonnement lumineux à travers la chambre de détection (23), ledit capteur (30) étant positionné de l'autre côté de ladite source par rapport à la zone de la chambre de détection.

6. Système selon l'une des revendications 3 à 5, **caractérisé en ce que** le support est constitué d'une carte micro-fluidique (20) réalisée dans un matériau transparent ou semi-transparent.

7. Système selon la revendication 6, **caractérisé en ce que** l'étage de dissolution ou de re-suspension comporte une ou plusieurs cuvettes (13) destinées chacune à recevoir un liquide (14) de dissolution ou de re-suspension.

8. Système selon la revendication 7, **caractérisé en ce que** le dispositif de préparation comporte un corps et **en ce que** l'étage de carottage comporte un ou plusieurs emporte-pièces (12) mobiles par rapport au corps, chaque emporte-pièce (12) coopérant en translation avec ledit corps suivant une direction principale (A), entre au moins un état de repos dans lequel l'emporte-pièce (12) est à l'aplomb d'une surface de dépôt (S) destiné à recevoir ledit prélèvement de matière et un état actionné dans lequel l'emporte-pièce est situé à l'intérieur d'une cuvette (13) distincte de l'état de dissolution ou de re-suspension.

9. Système selon la revendication 8, **caractérisé en ce que** chaque cuvette (13) comporte une ouverture et **en ce qu'**il comporte au moins un opercule (16) perforable recouvrant l'ouverture de chaque cuvette.

10. Système selon l'une des revendications 3 à 9, **caractérisé en ce que** le circuit fluidique comporte une zone de filtration des particules présentes dans l'échantillon liquide.

11. Système selon l'une des revendications 3 à 10, **caractérisé en ce que** le dispositif de lecture optique et l'unité de traitement sont regroupés dans un même dispositif de type téléphone intelligent.
